# EUROPEAN PATENT APPLICATION

(11) **EP 1 197 232 A1**
(43) Date of publication of application: **17.04.2002**
(21) Application number: 00950131.3
(22) Date of filing: 30.06.2000
(51) Int. Cl.: A61L 15/44

(54) **ABSORBENT ARTICLES OF PERSONAL HYGIENE WITH THERAPEUTIC EFFECT**

(30) Priority: 30.06.1999 RU 99113370
(71) Applicant: Zakrytoe Aktsionernoe Obschestvo Nauchno Proizvodstvennoe Meditsinskoe Predpriyatie "Bioekran", Moscow, 103051 (RU)
(72) Inventor: KOZINDA, Zinaida Julianovna, Moscow, 125080 (RU); SUVOROVA, Elena Grigorievna, Moscow, 105215 (RU); STRUKOV, Mikhail Vasilievich, Korolev Moskovskaya obl., 141070 (RU)
(74) Representative: Leach, John Nigel
(86) International application number: RU0000268
(87) International publication number: WO0100254

(57) **Abstract**

The invention relates to medical industry, namely, to means of private hygiene, including hygienic napkins and absorbing female pads of medicinal/prophylactic purpose.

An absorbing hygienic article having an antimicrobial substance contains Catamin and/or furagin as the antimicrobial substance, with the following component ratio, wt. %:

| | |
|---|---|
| Catamin | - 0.35-0.5 |
| Furagin | - 0-0.15 |

## Description

The present invention relates to medical industry, namely, to private hygiene means, including hygienic napkins and absorbing female pads of medicinal/prophylactic purpose.

At present, various types of absorbing hygienic pads are known, containing distributive, gel-forming, protective and other layers that are comfortable and have a purely hygienic function.

Physiological discharges are known to be an excellent nutritious medium for different types of bacteria and microorganisms whose development results in gynaecological diseases.

A non-woven material is currently known from the prior art used in the form of an article designed to collect discharges of human organism, in particular, disposable diapers, treated with linear polymeric biguanide to remove bacterial activity (see application EP 0174128, Cl. A 61 L 15/00, publ. 12.03.86).

The closest technical solution to the claimed invention is an absorbing fibrous material for absorbing physiological fluids impregnated before suspending with 250-270% aqueous solution comprising acrylic acid, boric acid, ammonium persulfate and sodium salt of 6-(paraaminobenzolsulfamide)-3-methoxypyridazine. Whereupon the material is exposed to UV radiation and dried. (USSR 1691440, Cl. A 61 F 13/15, publ. 15.11.91).

This material has insufficient activity against pathogenic microflora and insufficient lifetime during storage.

The object of the present invention is providing hygienic absorbing pads having antimicrobial properties for prophylactic and medicinal purposes.

This object is achieved by applying Catamin AB in mixture with Furagin to the cover layer with the following component ratio: (% of the weight of pad cover layer)

| | |
|---|---|
| Catamin AB | - 0.35-0.5 |
| Furagin | - 0-0.15 |

Catamin AB is alkyldimethylbenzylammonium chloride (synonym, Rockal) is an antiseptic and pertains to cationic surfactants. [R(CH₃)₂(CH₂C₆H₅)N]⁺Cl⁻ R-mixture of straight-chain alkyl groups C₁₀H₂₁-C₁₈H₃₇ or C₁₂H₂₅-C₁₄H₂₉. Medium molar weight of Catamin AB is 361±15 g/mole. Technical specifications TU 2482-012-1316 4401-94.

Furagin -N- (5-nitro-2furil) - allylidenaminohydantoin 02N CH=CH-CH=N-N.

These hygienic absorbing pads suppress microflora development, thereby enabling to protect the organism against its effect. They may be used both for medicinal and for prophylactic purposes. They do not induce allergy, mucosa irritation and do not affect the microflora balance existing within the organism.

As the cover layer of this article any type of the known materials can be used, the material in question being used in this field of medicine, namely, natural and synthetic fiberizing materials or mixtures thereof. The type of applying medicinal substances on the material may be different. This may be either, for example, active material absorption on the material, or medicinal material immobilization, etc.

The essence of the invention is illustrated with the following examples.

A non-woven material made from propylene fibers is impregnated in a bath containing an antimicrobial composition, Catamin AB in a concentration of 0.005-0.025% and furagin 0-0.0075%, the rest being water. Then follows pressing and drying. The area of microorganism growth inhibition and bath compositions are shown in the table.

Example No.1. A non-woven material made from propylene and cotton fibers is impregnated in a bath containing an antimicrobial composition, Catamin AB at a concentration of 0.005% and furagin 0.0075%, the rest being water. Then follows pressing and drying. Residual concentration of Catamin AB is 0.1% and of furagin, 0.15%. The area of staphylococcus growth inhibition on the material produced is 2.0 mm, that of colon bacillus is 1.0 mm and that of yeast fungi is 0.5 mm.

Example No.2. A cotton non-woven material is treated under the conditions described in Example No.1 at a Catamin AB concentration of 0.025%, the rest being water. Residual concentration of catamin AB after drying of the material is 0.5%. The area of staphylococcus growth inhibition on the material produced is 0.5 mm, that of colon bacillus is 0 mm, that of yeast fungi is 0.5 mm.

Example-No.3. A viscous material is treated under the conditions described in Example 1, at a Catamin AB concentration of 0.010% and furagin concentration of 0.005%, the rest being water. Residual concentration of Catamin AB is 0.2% and of furagin is 0.1%. The area of staphylococcus growth inhibition on the material produced is 1.5 mm, that of colon bacillus is 1.0 mm, that of yeast fungi is 1.0 mm.

Embodiments of the invention and properties of the material produced are provided in the table.

Antimicrobial properties in relation to different types of microorganisms are determined using the method of infected agar by the size of the growth inhibition area expressed in mm. Under medical/technical requirements, materials are considered antimicrobial when the size of the area of microorganism growth inhibition is not less than 2.0 mm.

Analysis of table data shows that the use of the proposed material having the antimicrobial coating provides growth inhibition of different types of microorganisms.

Volunteer patients who addressed medical institutions in connection with vaginal mucosa inflammation (vaginitis, vulvovaginitis, vulvitis) were invited to use, concurrently with the conventional treatment process, hygienic pads made from this material. 100 patients were observed. In none of them disease relapse was observed and convalescence was reduced by 1-2 days.

These hygienic articles, such as means of private hygiene, including hygienic napkins and absorbing female pads, possess antimicrobial activity and can be recommended as medlcinal/prophylactic.

**Table**

| Nº | catamin AB % | furagin % | water % | Area of inhibition of organism growth,mm | | |
|---|---|---|---|---|---|---|
| | | | | staphylococcus | colon bacillus | candida |
| propylene with cotton fibers 50:50 | | | | | | |
| for the method of producing antimicrobial material | | | | | | |
| 1 | 0,025 | - | 99,975 | 1,0 | 0 | 0,5 |
| 2 | 0,020 | 0,0005 | 99,9795 | 1,5 | 0,5 | 1,0 |
| 3 | 0,015 | 0,0025 | 99,825 | 2,0 | 1,0 | 1,5 |
| 4 | 0,01 | 0,005 | 99,985 | 2,0 | 1,5 | 1,5 |
| 5 | 0,005 | 0,0075 | 99,9875 | 2,0 | 1,0 | 0,5 |

| cotton material | | | | | | |
|---|---|---|---|---|---|---|
| composition and properties of antimicrobial material | | | | | | |
| 6 | 0,5 | - | | 0,5 | 0 | 0,5 |
| 7 | 0,4 | 0,01 | | 1,0 | 1,5 | 0,5 |
| 8 | 0,3 | 0,05 | | 2,0 | 2,5 | 2,0 |
| 9 | 0,2 | 0,10 | | 2,0 | 2,0 | 2,0 |
| 10 | 0,1 | 0,15 | | 1,5 | 1,0 | 1,0 |

| viscous material | | | | | | |
|---|---|---|---|---|---|---|
| 11 | 0,5 | - | | 0,5 | 0 | 0,5 |
| 12 | 0,4 | 0,01 | | 1,5 | 1.0 | 0,8 |
| 13 | 0,3 | 0,05 | | 2,0 | 2,0 | 2,0 |
| 14 | 0,2 | 0,10 | | 1,5 | 1,0 | 1,0 |
| 15 | 0,1 | 0,15 | | 1,0 | 1,0 | 1,0 |

| polyester material | | | | | | |
|---|---|---|---|---|---|---|
| 16 | 0,5 | - | | 1,0 | 0,5 | 0,7 |
| 17 | 0,4 | 0,01 | | 1,3 | 1,0 | 1,2 |
| 18 | 0,3 | 0,05 | | 2,0 | 2,0 | 2,3 |
| 19 | 0,2 | 0,10 | | 1,8 | 1,7 | 2,0 |
| 20 | 0,1 | 0,15 | | 1,0 | 1,0 | 1,2 |

| mixture of polypropylene and viscous fibers 50:50 | | | | | | |
|---|---|---|---|---|---|---|
| 21 | 0,5 | - | | 0,8 | 0,3 | 0,5 |
| 22 | 0,4 | 0,01 | | 1,3 | 1,0 | 1,2 |
| 23 | 0,3 | 0,05 | | 2,3 | 2,0 | 2,0 |
| 24 | 0,2 | 0,10 | | 2,0 | 2,1 | 1,6 |
| 25 | 0,1 | 0,15 | | 1,5 | 1,6 | 0,7 |

## Claims

1. An absorbing hygienic article having an antimicrobial substance, **characterised in that** it contains Catamin and/or furagin as the antimicrobial substance, with the following component ratio, wt. %:
| | |
|---|---|
| Catamin | - 0.35-0.5 |
| Furagin | - 0-0.15 |

2. An article according to claim 1, **characterized in that** it is formed as disposable hygienic female pads.

3. An article according to claim 1, **characterized in that** it is formed as hygienic napkins.
